Europäisches Patentamt

**European Patent Office**

**Office européen des brevets**

(11) Veröffentlichungsnummer: **0 349 952**

**A1**

(12) **EUROPÄISCHE PATENTANMELDUNG**

(21) Anmeldenummer: **89112111.3**

(51) Int. Cl.⁴: **G01N 1/14**

(22) Anmeldetag: **03.07.89**

(30) Priorität: **08.07.88 LU 87267**

(43) Veröffentlichungstag der Anmeldung:
**10.01.90 Patentblatt 90/02**

(84) Benannte Vertragsstaaten:
**BE DE ES FR GB GR IT LU NL**

(71) Anmelder: **EUROPÄISCHE ATOMGEMEINSCHAFT (EURATOM)**
**Bâtiment Jean Monnet Plateau du Kirchberg**
**L-2920 Luxembourg(LU)**

(72) Erfinder: **Van Loon, Leo**
**Via Lombardi 76**
**I-21029 Corgeno di Vergiate(IT)**
Erfinder: **Van Der Aat, René**
**Via Esperia 1**
**I-21020 Ispra(IT)**
Erfinder: **Mancinelli, Bruno**
**Via Torino 42**
**I-21021 Angera(IT)**
Erfinder: **Pilliard, Rémy**
**Via Lascina 35**
**Cuirone di Vergiate(IT)**
Erfinder: **Gaiardo, Marco**
**Via Bergamo 10**
**I-21020 Taino(IT)**

(74) Vertreter: **Weinmiller, Jürgen**
**Lennéstrasse 9 Postfach 24**
**D-8133 Feldafing(DE)**

(54) **Pneumatische Vorrichtung zur automatischen Entnahme einer Flüssigkeitsprobe aus einem Behälter.**

(57) Die Erfindung bezieht sich auf eine pneumatische Vorrichtung zur automatischen Entnahme einer Flüssigkeitsprobe aus einem Behälter (5) in eine Ampulle (9), die mit einem Gummideckel (8) verschlossen ist. Dabei ist die Probe durch eine Hohlnadel (7) ansaugbar, die mit dem Behälter (5) über eine Flüssigkeitsleitung (6) verbunden ist und den Deckel (8) durchstößt. Erfindungsgemäß besitzt die Vorrichtung einen pneumatisch anhebbaren Teller (10) zur Aufnahme einer Ampulle, wobei dem Teller gegenüber eine Saugnadel (14) liegt, die beim Anheben des Tellers in die Ampulle eindringt. Auf die Saugnadel gibt man wahlweise einen Überdruck zum Durchspülen der Leitungen oder einen Unterdruck zum Ansaugen einer Probe.

FIG. 1

## PNEUMATISCHE VORRICHTUNG ZUR AUTOMATISCHEN ENTNAHME EINER FLÜSSIGKEITSPROBE AUS EINEM BEHÄLTER

Die Erfindung bezieht sich auf eine pneumatische Vorrichtung zur automatischen Entnahme einer Flüssigkeitsprobe aus einem Behälter in eine Ampulle, die mit einem Gummideckel verschlossen ist, wobei die Probe durch eine Hohlnadel ansaugbar ist, die mit dem Behälter über eine Flüssigkeitsleitung verbunden ist und den Deckel durchstößt.

Bei der Bearbeitung von giftigen oder radioaktiven Flüssigkeiten müssen oft Proben an verschiedenen Stellen zu Analysezwekken entnommen werden. Erfolgt die Behandlung der Flüssigkeiten in unzugänglichen Räumen, sogenannten heißen Zellen, dann erfordern diese Probenentnahmen bisher komplizierte, von fernbedienten Manipulatoren betätigte Systeme und zwar an jeder Stelle, an der Proben entnommen werden sollen. Aufgabe der Erfindung ist es daher, eine einfache transportable Vorrichtung anzugeben, die auch mit nur grob wirkenden Manipulatoren zuverlässig bedient und an die verschiedenen Probeentnahmestellen transportiert werden kann, so daß nur eine einzige solche Vorrichtung für alle in einer heißen Zellen befindlichen Probeentnahmestellen vorgesehen werden muß.

Diese Aufgabe wird erfindungsgemäß mit einer pneumatischen Vorrichtung der eingangs genannten Art dadurch gelöst, daß die Vorrichtung einen pneumatisch anhebbaren Teller zur Aufnahme einer Ampulle besitzt, daß dem Teller gegenüber eine Saugnadel liegt, die beim Anheben des Tellers in die Ampulle eindringt, und daß die Saugnadel mit einem elektropneumatischen Ventil verbunden ist, das wahlweise einen Unterdruck oder einen Überdruck auf die Saugnadel gibt.

Es genügt daher für eine Probenentnahme, eine Ampulle auf den Teller zu setzen und den letzteren anzuheben, so daß die Saugnadel in die Ampulle eindringt. Dann wird eine Hohlnadel, die mit dem Behälter in Verbindung steht, aus dem eine Probe entnommen werden soll, ebenfalls durch den Deckel der Ampulle gestochen und die Vorrichtung eingeschaltet. Zuerst wird auf die Saugnadel ein Überdruck gegeben, so daß die Ampulle und über die Hohlnadel auch die Leitung in Richtung auf den Behälter mit Druckluft gespült wird. Dann erzeugt man in der Ampulle einen Unterdruck, mit dem die zu analysierende Flüssigkeit in die Ampulle gesaugt wird.

Vorzugsweise ist der Saugnadel eine Wasserstop-Klappe zugeordnet, die die Probenentnahme beendet, sobald Flüssigkeit anstatt Luft in die Saugnadel eindringt.

In einer bevorzugten Ausführungsform der erfindungsgemäßen Vorrichtung, wie sie in den Ansprüchen 2 und 3 definiert ist, wird der Unterdruck in einem Doppelzylinder erzeugt, dessen Energiequelle die Druckluftquelle ist. Auf diese Weise benötigt die Vorrichtung nur eine einzige Energiequelle für ihren Betrieb, nämlich die Druckluftquelle.

Nachfolgend wird die Erfindung anhand eines bevorzugten Ausführungsbeispiels mithilfe zweier Figuren näher erläutert.

Fig. 1 zeigt den schematischen Aufbau der ganzen Vorrichtung.

Fig. 2 zeigt einen Schnitt durch den pneumatischen Teil der Vorrichtung.

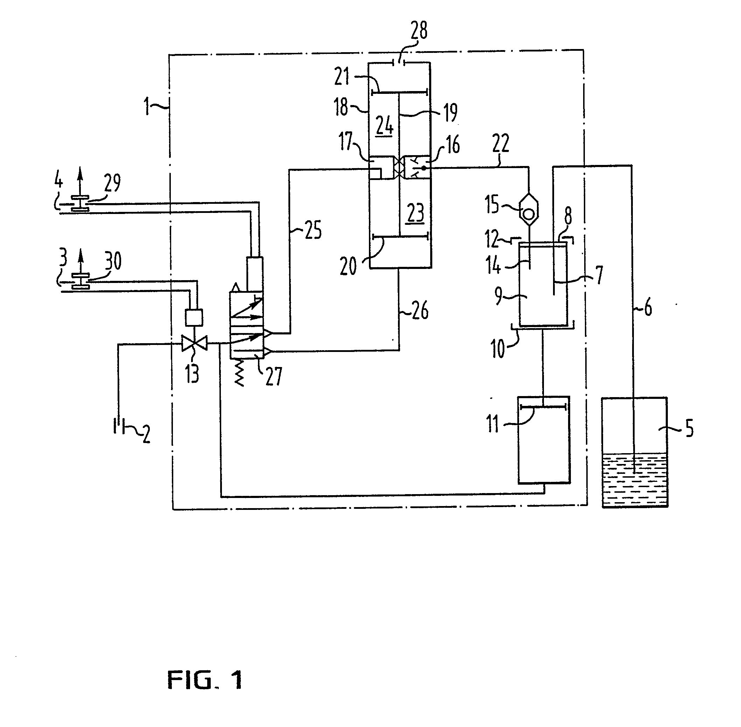

Die Vorrichtung ist in Fig. 1 durch eine strichpunktierte Linie 1 umschlossen und befindet sich in einer sogenannten heißen Zelle. Sie ist dort an eine Druckluftquelle 2 als einzige Energiequelle angeschlossen und im übrigen nur durch zwei elektrische Steuerleitungen 3 und 4 mit einem Steuerpult außerhalb der heißen Zelle verbunden. In dieser Zelle befindet sich auch ein Behälter 5, aus dem eine Probe entnommen werden soll. Der Behälter ist zu diesem Zweck mit einer in die Flüssigkeit des Behälters eintauchenden Leitung 6 versehen, die am äußeren Ende eine dünne Hohlnadel 7 trägt. Die Hohlnadel ist vorzugsweise ortsfest in der Nähe des Behälters 5 so montiert, daß ihre Spitze senkrecht nach unten zeigt und in einen dünnen Gummideckel 8 einer Ampulle 9 eindringen kann, wenn diese von unten an die Nadel angenähert wird. Es wäre aber auch umgekehrt möglich, die Vorrichtung in der Nähe des Behälters abzusetzen und die an einer flexiblen Leitung hängende Hohlnadel 7 mithilfe des Manipulators von oben in die Ampulle hineinzudrücken.

Die Ampulle 9 steht auf einem Teller 10, der von einem Pneumatikkolben 11 nach oben gegen einen Anschlag 12 schiebbar ist. Der Zylinder, in dem dieser Kolben 11 gleitet, ist über ein Haupt-Ventil 13 mit der Druckluftquelle 2 verbunden. Wird das Haupt-Ventil 13 geöffnet, dann hebt der Kolben 11 den Teller 10 und damit die Ampulle 9 an und schiebt letztere gegen den Anschlag 12. Dabei drückt sich eine ortsfest dem Teller gegenüberliegende Saugnadel 14 durch den Deckel 8 aus Gummi in die Ampulle hinein. Mithilfe einer elektropneumatischen Ventilanordnung wird durch die Saugnadel in die Ampulle wahlweise ein Überdruck eingespeist, um diese sowie die Leitung 6 durchzuspülen, oder ein Unterdruck erzeugt, um eine Probe aus dem Behälter 5 in die Ampulle anzusaugen. Diese Ventilanordnung wird nachfolgend erläutert.

Die Saugnadel ist über eine Wasserstop-Klappe 15 mit einer Dreiwege-Klappe 16 verbunden, die sich in einer Trennwand 17 eines Doppelzylinders 18 befindet. Die Trennwand 17 teilt den Doppelzylinder 18 in zwei Kammern 23 und 24 und wird von einer Kolbenstange 19 dicht durchdrungen, die an jedem Ende einen Kolben 20 und 21 trägt. Die Klappe 16 verbindet wahlweise die von der Wasserstop-Klappe 15 kommende Leitung 22 mit je einer Seite der Trennwand. Die erste Kammer 23 dieses Zylinders 18 wird durch den ersten Kolben 20 in zwei Räume geteilt, von denen der der Trennwand nahe Raum mit einer Druckluftleitung 25 verbunden ist, während der von der Trennwand entfernte Raum mit einer Druckluftleitung 26 verbunden ist. Die beiden Leitungen werden abwechselnd über ein Elektroventil 27 mit Druckluft versorgt, die von der Druckluftquelle 2 über das Hauptventil 13 geliefert wird. Die andere Kammer 24 ist stirnseitig mit einer Öffnung 28 versehen. Das Elektroventil 27 verbindet entweder die Leitung 26 mit der Druckluftquelle 2 und zugleich die Leitung 25 mit der Umgebungsluft der heißen Zelle, oder die Leitung 25 mit der Druckluftquelle 2 und gleichzeitig die Leitung 26 mit der Umgebungsluft.

Die von außerhalb der Zelle zu erteilenden Steuerbefehle sind durch Tasten 29 und 30 symbolisch angedeutet, die das Elektroventil 27 umschalten, bzw. das Drucklufthauptventil 13 öffnen oder schließen.

Die Vorrichtung gemäß Fig. 1 arbeitet folgendermaßen:

Soll eine Probe aus dem Behälter 5 entnommen werden, dann wird zuerst eine Ampulle 9 auf den von Hand oder mithilfe eines Manipulators nach unten gedrückten Teller 10 gestellt und dann das Druckluftventil 13 geöffnet. Dadurch wird der Teller 10 nach oben verschoben, und die Saugnadel 14 dringt durch den Gummideckel 8 der Ampulle in diese ein. Die Ampulle steht somit sicher in der Vorrichtung und wird gemeinsam mit dieser in die Nähe des Behälters 5 gebracht. Sie wird dann von unten so an die Hohlnadel 7 angenähert, daß letztere ebenfalls durch den Deckel 8 in die Ampulle eindringen kann.

Das Elektroventil 27 befindet sich währenddessen in der in der Figur gezeigten Stellung und drückt Druckluft über die Leitung 25 in die untere Kammer 23 des Doppelzylinders 18 hinein. Der Doppelkolben 20, 21 befindet sich in seiner unteren Stellung und drückt mit der Rückseite des oberen Kolbens 21 gegen einen (nicht dargestellten) Stößel, der die Dreiwegeklappe 16 so einstellt, daß die untere Kammer 23 über die Leitung 22 und die Wasserstop-Klappe 15 mit der Ampulle in Verbindung steht. Die durch die Leitung 25 gelieferte Druckluft dringt also durch die Leitung 22 in die Ampulle ein und gelangt durch die Hohlnadel 7 bis in den Behälter 5, aus dem die Probe entnommen werden soll. Damit wird in dieser Phase verhindert, daß giftige Gase aus dem Behälter in die Vorrichtung gelangen, und erreicht, daß die Hohlnadel 7 und die Leitung 6 durchgespült werden. Diese Position der Kolben kann als Bereitschaftsstellung der Vorrichtung bezeichnet werden. Die kann beliebig lang andauern.

Schaltet man nun das Elektroventil 27 um, dann wird statt der Leitung 25 die Leitung 26 mit Druckluft versorgt, so daß die beiden Kolben 20 und 21 in der Figur nach oben bewegt werden. Mit dieser Bewegung wird auch die Klappe 16 umgestellt, so daß nun die Leitung 22 mit der oberen Kammer 24 in Verbindung kommt, in der sich mit zunehmender Hubbewegung des Kolbens 21 ein Unterdruck einstellt. Der Unterdruck überträgt sich über die beiden Nadeln 14 und 7 auf die Behälterleitung 6, so daß in dieser Flüssigkeit aus dem Behälter nach oben steigt und sich in die Ampulle 9 ergießt. Das Hubvolumen des Kolbens 21 wird so bemessen, daß in jedem Fall eine ausreichende Menge der Flüssigkeit in die Ampulle gelangt.

Wenn das Flüssigkeitsniveau in der Ampulle die Saugnadel 14 erreicht, kann die Bedienungsperson die Druckluftversorgung über das Elektroventil 27 umschalten, so daß Kolben 20 und 21 sich wieder nach unten bewegen. Mit dieser Bewegung wird in der Kammer 24 ein Überdruck erzeugt, wobei über das Ventil 16 und die Leitung 22, Klappe 15 und Nadel 14 ein Überdruck in der Ampulle 9 bewirkt wird. Damit wird erreicht, daß die überflüssige Menge der Probeflüssigkeit wieder in den Behälter 5 zurückgedrückt wird. Das Gerät befindet sich wieder in der Bereitschaftsstellung (und "wäscht" die Nadel 7 und die Leitung 6!).

Wird durch die Bedienungsperson dagegen das Elektroventil 27 nicht umgeschaltet, dann wird die Wasserstop-Klappe 15 durch die steigende Probeflüssigkeit wirksam, die wiederum einen nicht dargestellten Mikroelektro-Kontakt schaltet. Dieser Kontakt betätigt dann das Elektroventil 27, womit der oben beschriebene Vorgang abläuft.

In beiden Fällen wird also vermieden, daß die Flüssigkeit in den Doppelzylinder 18 eindringt.

Die Bedienungsperson kann nun die Druckluftversorgung über das Ventil 13 ausschalten und die Hohlnadel 7 aus der Ampulle ziehen, indem sie die Vorrichtung nach unten entfernt, und dann die gesamte Vorrichtung zusammen mit der gefüllten Ampulle an anderer Stelle absetzen, wo der Teller 10 nach unten geschoben und die Ampulle 9 vom Teller entfernt werden kann.

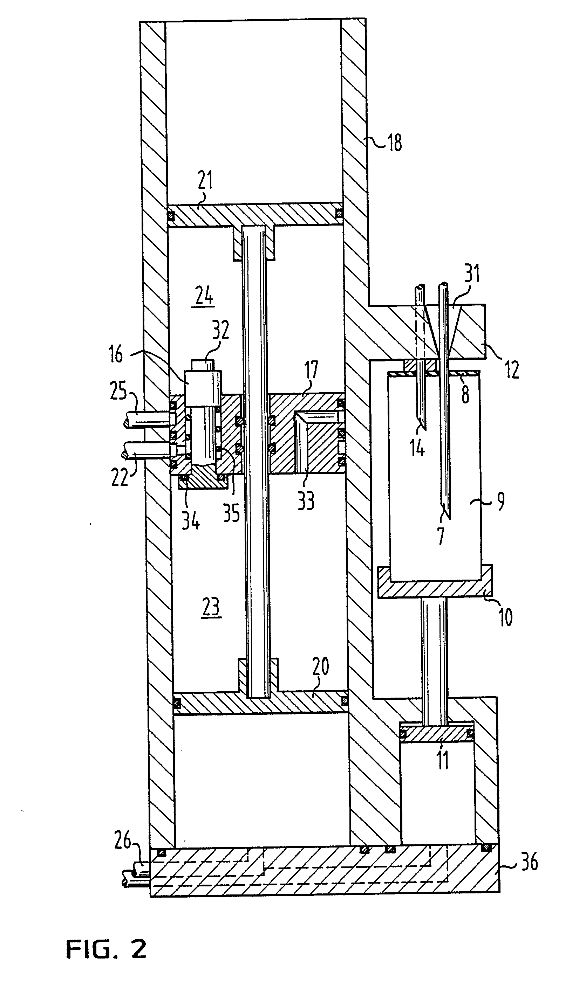

Die technische Ausführung der wesentlichen Bauteile der Vorrichtung geht aus der nun zu beschreibenden Figur 2 hervor. Dabei bezeichnen gleiche Bezugsziffern in beiden Figuren gleiche Bauteile. Der Kolben 11 zum Anheben des Tellers 10, auf dem die Ampulle 9 steht, ist baulich mit dem Doppelzylinder 18 vereint. Durch den gemeinsamen Boden 36 verlaufen nebeneinander sowohl die Druckluftleitung 26 als auch die Druckluftleitung zur Versorgung des Kolbens 11. In Fig. 2 ist eine Hohlnadel 7, die über eine Leitung mit einem hier nicht dargestellten Behälter verbunden ist, durch den Gummideckel 8 der Ampulle 9 in diese hineinragend dargestellt. Um das Einstecken dieser Hohlnadel zu erleichtern, ist oberhalb des Anschlags 12 ein Führungstrichter 31 vorgesehen. Die Ampulle ist in Bereitschaftsstellung dargestellt, d.h., daß die Saugnadel 14 in die Ampulle hineinragt und die Ampulle am Anschlag 12 anliegt.

Die beiden Kolben 20 und 21 im Doppelzylinder 18 sind in einer mittleren Stellung dargestellt. In der Bereitschaftsstellung liegt der Kolben 21 auf einem Stößel 32 der Klappe 16 auf und drückt diese in eine Stellung, in der ein mit der Leitung 22 verbundener Ringraum mit der unteren Kammer 23 in Verbindung steht. Zugleich steht mit derselben Kammer die Leitung 25 über einen dazu parallelen Ringraum und einen Kanal 33 innerhalb der Trennwand 17 in Verbindung.

In der in Fig. 2 gezeigten Kolbenzwischenstellung ist die Klappe 16 in Richtung auf die Kammer 23 geschlossen, da ein Klappenkopf mit seiner Ringdichtung 34 unter der Wirkung einer Rückstellfeder 35 auf der Trennwand aufliegt. Der sich bei der Aufwärtsbewegung des Kolbens 21 ausbildende Unterdruck im unteren Raum der der Kammer 24 wird nun auch in der Leitung 22 und in der Saugnadel 14 wirksam.

Die beiden Elektroventile 13 und 27 sind hier nicht dargestellt, da es sich um konventionelle Bauteile handelt, die an geeigneter Stelle an die in Fig. 2 gezeigte Vorrichtung angebaut sind. Ebenso sind auch die an einen Manipulator angepaß ten Greifbacken in der Figur nicht dargestellt, an denen der Manipulator die Vorrichtung ergreifen und so bezüglich der Hohlnadel eines Behälters 5 in Stellung bringen kann, daß diese Hohlnadel 7 in die Ampulle eindringt.

Der Füllgrad der Ampulle ergibt sich automatisch aufgrund der gegebenenfalls veränderbaren Stellung der Saugnadel 14, und zwar unabhängig von der von Probenentnahmestelle zu Probenentnahmestelle unterschiedlichen Länge der Leitung 6 zwischen der Hohlnadel 7 und dem Behälter 5.

An der ganzen Vorrichtung bestehen nur zwei Steuermöglichkeiten, nämlich das Einschalten des Druckluftversorgungsventils 13 und das Umschalten des Elektroventils 27. Eine Fehlbedienung ist damit praktisch ausgeschlossen. Fällt die Druckluft als einzige Energiequelle aus, dann wird sofort der Saugvorgang unterbrochen, aber die bereits angesaugte Probenmenge verbleibt sicher in der Ampulle.

Die Erfindung ist nicht auf das oben erläuterte bevorzugte Ausführungsbeispiel beschränkt. So kann insbesondere der Doppelzylinder entfallen, wenn getrennte Quellen für Druckluft und Unterdruck zur Verfügung stehen. Das Ventil 27 besorgt dann alleine die Umschaltung zwischen diesen beiden Quellen.

## Ansprüche

1. Pneumatische Vorrichtung zur automatischen Entnahme einer Flüssigkeitsprobe aus einem Behälter in eine Ampulle, die mit einem Gummideckel verschlossen ist, wobei die Probe durch eine Hohlnadel ansaugbar ist, die mit dem Behälter über eine Flüssigkeitsleitung verbunden ist und den Deckel durchstößt, dadurch **gekennzeichnet,** daß die Vorrichtung einen pneumatisch anhebbaren Teller (10) zur Aufnahme einer Ampulle (9) besitzt, daß dem Teller gegenüber eine Saugnadel (14) liegt, die beim Anheben des Tellers in die Ampulle eindringt und daß die Saugnadel mit einer elektropneumatischen Ventilanordnung (18-21, 27) verbunden ist, die wahlweise einen Unterdruck oder einen Überdruck auf die Saugnadel gibt.

2. Vorrichtung nach Anspruch 1, dadurch gekennzeichnet, daß die elektropneumatische Ventilanordnung einen Doppelzylinder (18) aufweist, dessen beide Kolben (20, 21) in je einer Kammer (23, 24) beweglich angeordnet sind, wobei die die beiden Kolben verbindende Kolbenstange (19) eine Trennwand (17) zwischen den Kammern dicht durchdringt, daß in die erste Kammer (23) im Bereich der Trennwand und im Bereich der Stirnwand je eine Druckluftleitung (25, 26) mündet, während die andere Kammer (24) stirnseitig offen ist, daß die Trennwand eine Dreiwege-Klappe (16) aufweist, die eine zur Saugnadel (14) führende Leitung (22) mit der ersten Kammer (23) verbindet, wenn der Kolben in der anderen Kammer (24) gegen die Trennwand (17) anliegt, während ansonsten die zur Saugnadel (14) führende Leitung (22) mit der anderen Kammer (24) in Verbindung steht.

3. Vorrichtung nach Anspruch 2, dadurch gekennzeichnet, daß die beiden Druckluftleitungen (25, 26) mit einem Elektroventil 27) verbunden sind, das abwechselnd Druckluft auf diese Lei tungen gibt, wobei die gerade nicht mit Druckluft versorgte Leitung im Elektroventil (27) mit der Umgebungsluft verbunden wird.

4. Vorrichtung nach einem der Ansprüche 1 bis 3, dadurch gekennzeichnet, daß die Saugnadel (14) mit der elektropneumatischen Ventilanordnung (18-21, 27) über eine Wasserstop-Klappe (15) verbunden ist, die verhindert, daß Flüssigkeit durch die Saugnadel angesaugt wird.

FIG. 1

EP 0 349 952 A1

FIG. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | FR-A-1 568 072 (COMPAGNIE GENERALE D'AUTOMATISME) <br> * Seite 1, Spalte 2, Zeile 35 - Seite 3, Spalte 2, Zeile 37; Figur 1 * <br> --- | 1 | G 01 N 1/14 |
| X | US-A-3 872 730 (M.A. RINGROSE et al.) <br> * Spalte 2, Zeile 36 - Spalte 5, Zeile 54; Figuren 1-6 * <br> --- | 1 | |
| A | DE-A-2 824 155 (FA. E. BÜHLER) <br> * Seite 9, Zeile 27 - Seite 18, Zeile 25; Figuren 1-5 * <br> --- | 1,2 | |
| A | FR-A-2 400 197 (MKT-TEHTAAT OY) <br> * Insgesamt * <br> --- | 1 | |
| A | EP-A-0 155 107 (BRITISH NUCLEAR FUELS PLC) <br> * Seite 2, Zeile 4 - Seite 4, Zeile 9; Figuren 1,2 * <br> ----- | 1 | RECHERCHIERTE SACHGEBIETE (Int. Cl.5) <br><br> G 01 N 1/00 <br> B 01 L 11/00 <br> G 02 F 5/00 <br> G 02 F 17/00 |

Der vorliegende Recherchenbericht wurde für alle Patentansprüche erstellt

| Recherchenort | Abschlußdatum der Recherche | Prüfer |
|---|---|---|
| DEN HAAG | 11-08-1989 | SARNEEL A.P.T. |